# EUROPEAN PATENT APPLICATION

(11) **EP 4 616 786 A1**
(43) Date of publication of application: **17.09.2025**
(21) Application number: 24869445.7
(22) Date of filing: 01.02.2024
(51) Int. Cl.: A61B 1/317, A61B 17/94

(54) **SPINAL ENDOSCOPE WITH LOCALIZABLE WORKING SLEEVE**

(71) Applicant: Peking University Third Hospital (Peking University Third School of Clinical Medicine), Haidian District Beijing 100191 (CN); Shanghai Reach Medical Instrument Co.,Ltd, Shanghai 201114 (CN)
(72) Inventor: LI, Weishi, Shanghai 201114 (CN); HUANG, Xiaomin, Shanghai 201114 (CN); ZHONG, Woquan, Shanghai 201114 (CN); JIANG, Shuai, Shanghai 201114 (CN); YU, Mingpeng, Shanghai 201114 (CN); YANG, Xing, Shanghai 201114 (CN)
(74) Representative: Valet Patent Services Limited
(86) International application number: PCT/CN2024/075248
(87) International publication number: WO 2025/160891

(57) **Abstract**

The invention discloses a spinal endoscope positionable with a working sleeve, including a transforaminal endoscope assembly, a working sleeve movably connected with the transforaminal endoscope assembly, and a positioning connecting assembly used for fixing the transforaminal endoscope assembly and the working sleeve; wherein the transforaminal endoscope assembly is provided with a fixed block through which an endoscope pipeline penetrates; a handpiece is fixedly connected to a periphery, close to the transforaminal endoscope assembly, on the working sleeve, an end, away from the transforaminal endoscope assembly, of the endoscope pipeline extends into the working sleeve, and at the same time, an outer arc surface of the endoscope pipeline is fitted to an inner surface of the working sleeve; and the positioning connecting assembly is fixedly connected to the endoscope pipeline, when the positioning connecting assembly is pressed, the working sleeve is movable along a length direction of the endoscope pipeline, and when the working sleeve is adjusted to an appropriate position, the positioning connecting assembly is released to fix the working sleeve. According to the invention, a position of a transforaminal endoscope in the working sleeve is adjusted, which facilitates positioning; and the working sleeve and the transforaminal endoscope are assembled while being disassembled, which facilitates cleaning and disinfection. Due to surface fitting, the transforaminal endoscope occupies the minimum working space and retains the maximum operation space of a working channel, thereby facilitating surgical operation.

## Description

### Technical Field

The invention relates to the technical field of spinal endoscopes, in particular to a spinal endoscope positionable with a working sleeve.

### Background

An MISS (minimal invasive spine surgery) technology has been rapidly developed and has been widely applied and recognized in the industry, thereby bringing good news to millions of patients with spinal diseases around the world. The technology will lead the trend of future development in the field of spinal surgery. The MISS technology requires spinal surgeons to complete an entire surgical process by means of a surgical incision as small as possible by applying special surgical instruments and apparatuses with the assistance of imaging devices, thereby achieving the purposes of reducing the incisions, reducing tissue trauma and bleeding, improving the operation accuracy and efficacy, and promoting postoperative recovery. However, a working sleeve and a transforaminal endoscope assembly in an existing spinal endoscope cannot be assembled while being disassembled, and at the same time, a diameter of a working channel is very large, which causes large incisions and great trauma to a human body.

### Summary of the Invention

For defects existing in the prior art, the invention aims to provide a spinal endoscope positionable with a working sleeve. A position of a transforaminal endoscope in the working sleeve is adjusted, which facilitates positioning; and the working sleeve and the transforaminal endoscope are assembled while being disassembled, which facilitates cleaning and disinfection. The endoscope is perfectly fitted to the working sleeve, so that the maximum operation space for a surgical tool is achieved. In order to achieve the above-mentioned objects and other advantages according to the invention, provided is a spinal endoscope positionable with a working sleeve, including:
a transforaminal endoscope assembly, a working sleeve movably connected with the transforaminal endoscope assembly, and a positioning connecting assembly used for fixing the transforaminal endoscope assembly and the working sleeve; wherein
the transforaminal endoscope assembly is provided with a fixed block through which an endoscope pipeline penetrates;
a handpiece is fixedly connected to a periphery, close to the transforaminal endoscope assembly, on the working sleeve, and an end, away from the transforaminal endoscope assembly, of the endoscope pipeline extends into the working sleeve; and
the positioning connecting assembly is fixedly connected to the endoscope pipeline, when the positioning connecting assembly is pressed, the working sleeve is movable along a length direction of the endoscope pipeline, and when the working sleeve is adjusted to an appropriate position, the positioning connecting assembly is released to fix the working sleeve. A part, inserted into the working sleeve, of the endoscope pipeline is fitted to an inner surface of the working sleeve.

Preferably, the positioning connecting assembly includes a transforaminal endoscope and fixed connection sleeve, a positioning cross bar fixed on the fixed block, a button structure sleeved on the positioning cross bar, and an elastic part fixed on the button structure, one end of the elastic part is fixedly connected to the button structure, and the other end thereof is in mutual contact with the endoscope pipeline.

Preferably, the button structure includes a button part and a sleeving block fixedly connected with the button part, a through hole is formed in the middle of the sleeving block, the positioning cross bar penetrates through the through hole, an end face, away from the button part, of the sleeving block is fixedly connected with the elastic part, and the sleeving block is fixedly connected with a positioning pin at the elastic part.

Preferably, a plurality of positioning holes distributed uniformly are formed in a length direction of the positioning cross bar; and the positioning pin is located on the sleeving block and extends to the button part, so that one end of the positioning pin is flush with a surface of the sleeving block, the other end thereof is located in the through hole, and the positioning pin is matched with the positioning holes.

Preferably, one end of the positioning cross bar is fixedly connected with the fixed block, the other end thereof is spaced from the handpiece, and the button structure moves along the length direction of the positioning cross bar.

Preferably, the elastic part is two springs, and when a force is applied to the elastic part, a distance value generated when the elastic part deforms is the same as an extension length of the positioning pin in the through hole.

Compared with the prior art, the invention has the beneficial effects that by pressing the button part, the positioning pin can be separated from the through hole in the positioning cross bar, at the moment, the working sleeve can be moved to a required position; then, the button part is released, at the moment, the positioning pin can be inserted into another through hole in the positioning cross bar to position and lock the working sleeve; and after use, by pressing the button part all the time, the working sleeve and the transforaminal endoscope can be disassembled, which facilitates disinfection and cleaning.

### Brief Description of the Drawings

FIG.1 is a schematic diagram of a three-dimensional structure of a spinal endoscope positionable with a working sleeve according to the invention;
FIG. 2 is a schematic diagram of a three-dimensional structure, with a transforaminal endoscope and fixed connection sleeve being removed, of a spinal endoscope positionable with a working sleeve according to the invention;
FIG. 3 is a schematic diagram of a specific structure of a positioning connecting assembly of a spinal endoscope positionable with a working sleeve according to the invention;
FIG. 4 is a schematic diagram of a three-dimensional handpiece of a transforaminal endoscope and fixed connection sleeve of a spinal endoscope positionable with a working sleeve according to the invention; and
FIG. 5 is a schematic diagram of a three-dimensional structure, with a working sleeve being removed, of a spinal endoscope positionable with the working sleeve according to the invention.

### Detailed Description of the Preferred Embodiments

Technical solutions in the embodiments of the invention will be described clearly and completely below in conjunction with the accompanying drawings in the embodiments of the invention. Obviously, the described embodiments are only a part of the embodiments of the invention, not all the embodiments. Based on the embodiments in the invention, all other embodiments obtained by those of ordinary skill in the art without creative work shall fall within the protection scope of the invention.

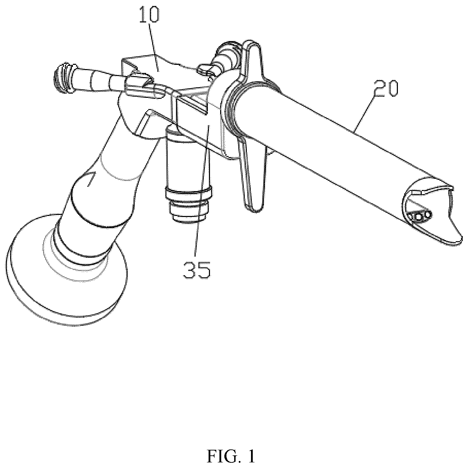

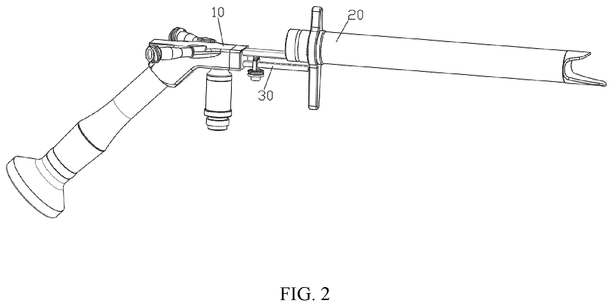

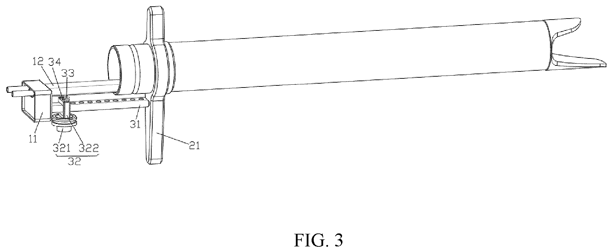

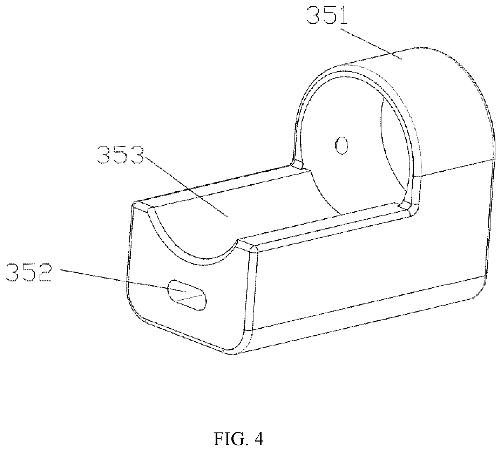

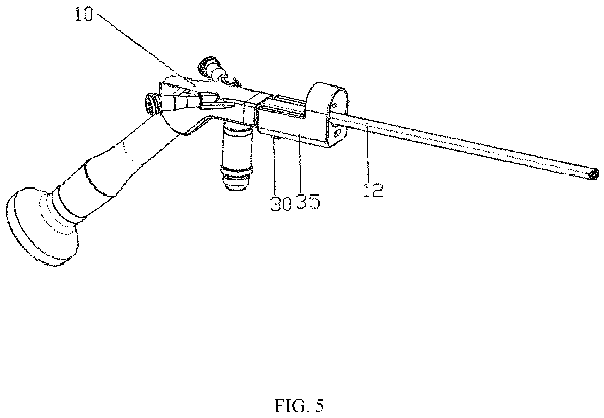

Referring to FIGS. 1 to 5, provided is a spinal endoscope positionable with a working sleeve, including a transforaminal endoscope assembly 10, a working sleeve 20 movably connected with the transforaminal endoscope assembly 10, and a positioning connecting assembly 30 used for fixing the transforaminal endoscope assembly 10 and the working sleeve 20; wherein an outer wall of a transforaminal endoscope in the transforaminal endoscope assembly 10 is fitted to an inner wall of the working sleeve 20, so that the transforaminal endoscope assembly 10 will have the maximum operation space for a surgical tool during work; the transforaminal endoscope assembly 10 is provided with a fixed block 11 through which an endoscope pipeline 12 penetrates; a handpiece 21 is fixedly connected to a periphery, close to the transforaminal endoscope assembly 10, on the working sleeve 20, and an end, away from the transforaminal endoscope assembly 10, of the endoscope pipeline 12 extends into the working sleeve 20; and the positioning connecting assembly 30 is fixedly connected to the endoscope pipeline 12, when the positioning connecting assembly 30 is pressed, the working sleeve 20 is movable along a length direction of the endoscope pipeline 12, and when the working sleeve 20 is adjusted to an appropriate position, the positioning connecting assembly 30 is released to fix the working sleeve 20. A distance between the working sleeve 20 and the transforaminal endoscope assembly 10 is adjusted by the positioning connecting assembly 30, so that it is convenient for a doctor to adjust a position of the endoscope in the working sleeve 20 during a surgery.

Further, the positioning connecting assembly 30 includes a transforaminal endoscope and fixed connection sleeve 35, a positioning cross bar 31 fixed on the fixed block 11, a button structure 32 sleeved on the positioning cross bar 31, and an elastic part 33 fixed on the button structure 32, wherein the transforaminal endoscope and fixed connection sleeve 35 includes an annular sleeve 315 used for sleeving the working sleeve 20 and an arc-shaped channel 353 used for extending the endoscope pipeline 12, a through splicing hole 352 is formed in the middle of the transforaminal endoscope and fixed connection sleeve 35, the positioning cross bar 31 is interspersed in the splicing hole 352, a mounting hole used for mounting the button structure 32 is formed in a part, close to a button part 321, of the transforaminal endoscope and fixed connection sleeve 35, the transforaminal endoscope and fixed connection sleeve 35 is disposed between the working sleeve 20 and the transforaminal endoscope assembly 10 and is used for fixing the working sleeve 20, when the working sleeve 10 moves, the transforaminal endoscope and fixed connection sleeve 35 is driven to move, one end of the elastic part 33 is fixedly connected to the button structure 32, and the other end thereof is in mutual contact with the endoscope pipeline 12. The button structure 32 includes the button part 321 and a sleeving block 322 fixedly connected with the button part 321, a through hole is formed in the middle of the sleeving block 322, the positioning cross bar 31 penetrates through the through hole, an end face, away from the button part 321, of the sleeving block 322 is fixedly connected with the elastic part 33, and the sleeving block 322 is fixedly connected with a positioning pin 34 at the elastic part 33. A plurality of positioning holes distributed uniformly are formed in a length direction of the positioning cross bar 31; and the positioning pin 34 is located on the sleeving block 322 and extends to the button part 321, so that one end of the positioning pin 34 is flush with a surface of the sleeving block 322, the other end thereof is located in the through hole, and the positioning pin 34 is matched with the positioning holes. When a position of the endoscope needs to be adjusted, the positioning pin 321 moves out of the through hole of the positioning cross bar 31 by pressing the button part 321, at the moment, the button part 321 is pressed without moving, the transforaminal endoscope assembly 10 moves to the working sleeve 20 to reach a position required by a surgery, then, the button part 321 is released, at the moment, the positioning pin 34 is aligned with the corresponding positioning hole in the positioning cross bar 31, the distance between every two positioning holes is the same, sequential position gears are correspondingly adjusted every time when the positioning pin 34 moves for a position of one positioning hole, a plurality of positioning holes can be skipped by one-time movement, and when the positioning pin 34 falls into the positioning hole, the position of the working sleeve 20 is locked.

Further, one end of the positioning cross bar 31 is fixedly connected with the fixed block 11, the other end thereof is spaced from the handpiece 21, and the button structure 32 moves along the length direction of the positioning cross bar 31.

Further, the elastic part 33 is two springs, and when a force is applied to the elastic part 33, a distance value generated when the elastic part 33 deforms is the same as an extension length of the positioning pin 34 in the through hole, so that the positioning pin 34 can be separated from the through hole when the button part 321 is pressed, which facilitates the displacement of the working sleeve 20.

The number of devices and the treatment scale described herein are intended to simplify the description of the invention, and the application, modification and change of the invention are obvious to the skilled in the art.

Although the embodiments of the invention have been disclosed as above, the invention is not limited to applications listed in the specification and implementations and is completely applicable to various fields suitable for the invention. For those skilled in the art, it is easy to achieve additional modifications. Therefore, when not departing from the general concept limited by the claims and an equivalent scope thereof, the invention is not limited to specific details and figures shown and described herein.

## Claims

1. A spinal endoscope positionable with a working sleeve, **characterized by** comprising:
a transforaminal endoscope assembly (10), a working sleeve (20) movably connected with the transforaminal endoscope assembly (10), and a positioning connecting assembly (30) used for fixing the transforaminal endoscope assembly (10) and the working sleeve (20); wherein
the transforaminal endoscope assembly (10) is provided with a fixed block (11) through which an endoscope pipeline (12) penetrates;
a handpiece (21) is fixedly connected to a periphery, close to the transforaminal endoscope assembly (10), on the working sleeve (20), an end, away from the transforaminal endoscope assembly (10), of the endoscope pipeline (12) extends into the working sleeve (20), and a part, inserted into the working sleeve (20), of the endoscope pipeline (12) is fitted to an inner surface of the working sleeve (20); and
the positioning connecting assembly (30) is fixedly connected to the endoscope pipeline (12), when the positioning connecting assembly (30) is pressed, the working sleeve (20) is movable along a length direction of the endoscope pipeline (12), and when the working sleeve (20) is adjusted to an appropriate position, the positioning connecting assembly (30) is released to fix the working sleeve (20).

2. The spinal endoscope positionable with the working sleeve according to claim 1, **characterized in that** the positioning connecting assembly (30) comprises a transforaminal endoscope and fixed connection sleeve (35), a positioning cross bar (31) fixed on the fixed block (11), a button structure (32) sleeved on the positioning cross bar (31), and an elastic part (33) fixed on the button structure (32), one end of the elastic part (33) is fixedly connected to the button structure (32), and the other end thereof is in mutual contact with the endoscope pipeline (12).

3. The spinal endoscope positionable with the working sleeve according to claim 2, **characterized in that** the button structure (32) comprises a button part (321) and a sleeving block (322) fixedly connected with the button part (321), a through hole is formed in the middle of the sleeving block (322), the positioning cross bar (31) penetrates through the through hole, an end face, away from the button part (321), of the sleeving block (322) is fixedly connected with the elastic part (33), and the sleeving block (322) is fixedly connected with a positioning pin (34) at the elastic part (33).

4. The spinal endoscope positionable with the working sleeve according to claim 3, **characterized in that** a plurality of positioning holes distributed uniformly are formed in a length direction of the positioning cross bar (31); and the positioning pin (34) is located on the sleeving block (322) and extends to the button part (321), so that one end of the positioning pin (34) is flush with a surface of the sleeving block (322), the other end thereof is located in the through hole, and the positioning pin (34) is matched with the positioning holes.

5. The spinal endoscope positionable with the working sleeve according to claim 4, **characterized in that** one end of the positioning cross bar (31) is fixedly connected with the fixed block (11), the other end thereof is spaced from the handpiece (21), and the button structure (32) moves along the length direction of the positioning cross bar (31).

6. The spinal endoscope positionable with the working sleeve according to claim 5, **characterized in that** the elastic part (33) is two springs, and when a force is applied to the elastic part (33), a distance value generated when the elastic part (33) deforms is the same as an extension length of the positioning pin (34) in the through hole.
